(19) Europäisches Patentamt
European Patent Office
Office européen des brevets

(11) EP 1 215 996 B1

## (12) EUROPEAN PATENT SPECIFICATION

(45) Date of publication and mention
of the grant of the patent:
**24.11.2004 Bulletin 2004/48**

(21) Application number: **00967099.3**

(22) Date of filing: **29.09.2000**

(51) Int Cl.⁷: $A61B\ 5/0452$, $G06F\ 17/00$

(86) International application number:
**PCT/US2000/026822**

(87) International publication number:
**WO 2001/022878 (05.04.2001 Gazette 2001/14)**

(54) **ANALYTICAL SIGNAL METHOD FOR ANALYSIS OF T-WAVE ALTERNANS**

VERFAHREN ZUR ANALYSE VON T-ZACKEN MITTELS ANALYTISCHEN SIGNALEN

PROCEDE AXE SUR LE SIGNAL ANALYTIQUE POUR L'ANALYSE DE L'ALTERNANCE DES ONDES T

(84) Designated Contracting States:
**DE GB IT NL**

(30) Priority: **29.09.1999 US 156524 P**

(43) Date of publication of application:
**26.06.2002 Bulletin 2002/26**

(73) Proprietor: **CAMBRIDGE HEART, INC.
Bedford, MA 01730 (US)**

(72) Inventor: **HAGHIGHI-MOOD, Ali
Andover, MA 01810 (US)**

(74) Representative: **Howe, Steven
Lloyd Wise
Commonwealth House,
1-19 New Oxford Street
London WC1A 1LW (GB)**

(56) References cited:
US-A- 4 802 491    US-A- 5 570 696
US-A- 5 704 365

- **S L MARPLE JR: "Computing the Discrete-Time 'Analytic' Signal Via FFT" ASILOMAR CONFERENCE ON SIGNALS, SYSTEMS AND COMPUTERS,US,LOS ALAMITOS, CA.: IEEE, 2 November 1997 (1997-11-02), pages 1322-1325, XP002123670 ISBN: 0-8186-8317-1**
- **FOKAPU O ET AL: "A NEW APPROACH FOR P WAVE DETECTION USING ANALYTIC SIGNAL" PROCEEDINGS OF THE ANNUAL INTERNATIONAL CONFERENCE OF THE ENGINEERING IN MEDICINE AND BIOLOGY SOCIETY,US,NEW YORK, 1EEE, vol. CONF. 15, 28 October 1993 (1993-10-28), pages 400-401, XP000436800**
- **MURDA'H M A ET AL: "REPOLARIZATION ALTERNANS: TECHNIQUES, MECHANISMS, AND CARDIAC VULNERABILITY" PACE - PACING AND CLINICAL ELECTROPHYSIOLOGY,FUTURA PUBLISHING COMPANY, INC,US, vol. 20, no. 10, PART 02, 1 October 1997 (1997-10-01), pages 2641-2657, XP000723368 ISSN: 0147-8389**

## Description

The invention is directed to techniques for measuring alternans in an electrocardiogram (ECG) waveform.

Alternans, a subtle beat-to-beat change in the repeating pattern of an (ECG) waveform can be indicative of electrical instability of the heart and increased susceptibility to sudden cardiac death. Alternans results in an ABABAB... pattern of variation of waveform shape between successive beats in an ECG waveform. The level of variation has been found to be a useful characterization of an individual's cardiac electrical stability, with increasing variation being indicative of decreasing stability.

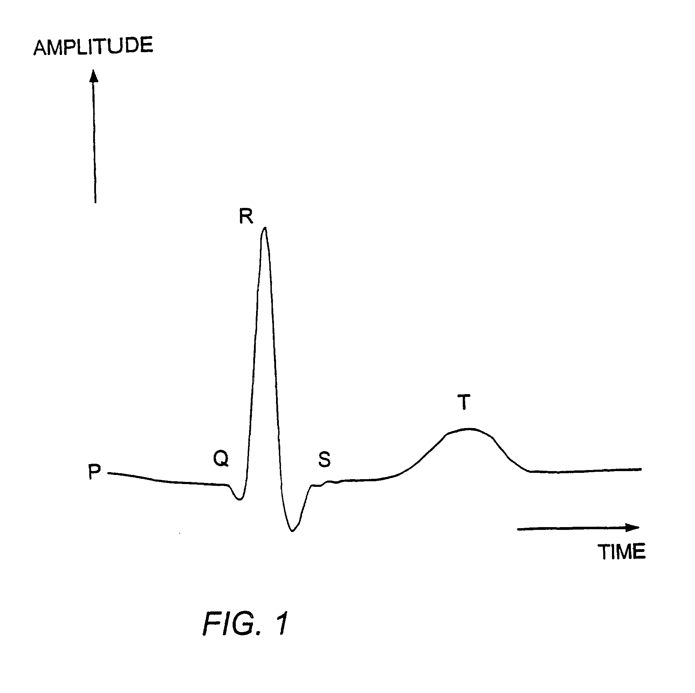

Referring to Fig. 1, an ECG waveform for a single beat is typically referred to as a PQRST complex. Briefly, the P wave appears at initiation of the beat and corresponds to activity in the atria, while the QRST complex follows the P wave and corresponds to ventricular activity. The QRS component represents the electrical activation of the ventricles, while the T wave represents their electrical recovery. The ST segment is a relatively quiescent period. It has been found that the T wave is the best interval of the ECG complex for detecting alternans. That is, a level of variation in the T waves of alternating beats is the best indicator of the electrical stability of the ventricles - the heart's main pumping chambers.

While an ECG waveform typically has a QRS amplitude measured in millivolts, an alternans pattern of variation with an amplitude on the order of a microvolt may be clinically significant. Accordingly, the alternans pattern may be too small to be detected by visual inspection of the electrocardiogram and often must be detected and quantified electronically. Such electronic detection and quantification of the alternans pattern is further complicated by the presence of noise in the ECG waveforms, as the noise may result in beat-to-beat variations that have a larger magnitude than the alternans pattern of variation.

The noise in an ECG signal can be classified into three categories: baseline noise generated in the electrode, physiologic noise, and external electrical noise. The baseline noise is low frequency noise that appears as an undulating baseline upon which the ECG rides. Baseline noise is attributable to motion and deformation of the electrode, and results from low frequency events such as patient respiration and patient motion. As a result, the magnitude of baseline noise tends to increase with exercise. Typically, the frequency content of baseline noise is below 2 Hz.

Physiologic noise results from physiologic processes, such as skeletal muscle activity, that interfere with the ECG signal. The electrical activity of the skeletal muscles creates potentials that are additive with respect to the potentials created by the heart. The frequency content of the skeletal muscle signals is comparable to the frequency content of the QRS complex, and is typically greater than 10 Hz. When measuring T-wave alternans, additional physiologic noise may result from changes in the position of the heart due to respiration or from changes in the projection of the electrical potential from the heart to the skin surface due to thoracic conductivity changes arising from the inflation and deflation of the lungs with respiration.

External electrical noise results, for example, from ambient electromagnetic activity in the room, electrode cable motion, and variations in amplifiers or other components of the ECG circuitry. External electrical noise may be eliminated or reduced through the use of high quality components and through the reduction of ambient electromagnetic activity by, for example, deactivating high power equipment.

Noise in the ECG waveform also can mimic the presence of alternans where none exists. For example, if a patient is breathing at one half or one third of the heart rate, the respiration may introduce a harmonic signal having the ABABAB... pattern of alternans. Motion that repeats with some periodicity, such as that resulting from exercise, can create electrode noise with a similar pattern.

One alternans measurement approach that attempts to address the effects of noise is referred to as the spectral method for measuring T-wave alternans. This method is described in detail in U.S. patent 4,802,491. In summary, this method involves concurrently analyzing 128 beats of a continuous stream of ECG signals. The spectral method uses measurements from time synchronized points of consecutive T waves. A time series is created by measuring, for each of the 128 beats, the T-wave level at a fixed point relating to the QRS complex. This process is repeated to create a time series for each point in the T wave. A frequency spectrum is then generated for each time series, and the spectra are averaged to form a composite T-wave alternans spectrum. Since the T-waves are sampled once per beat for each time series, the spectral value at the Nyquist frequency, i.e. 0.5 cycle per beat, indicates the level of beat-to-beat alternation in the T-wave waveform.

The alternans power is statistically compared to the noise power to discriminate the beat-to-beat T-wave variation due to abnormal electrical activity of the heart from the random variation due to background noise. The alternans power is calculated by subtracting the mean power in a reference band used to estimate the background noise level (for example, the frequency band of 0.44-0.49 cycle per beat) from the power at the Nyquist frequency (0.50 cycle per beat). Alternans is considered to be significant if the alternans is at least three times the standard deviation of the noise in the noise reference band.

US Patent No. 5,704,365 describes a method for reducing noise in a signal that represents a physiological process. The method includes obtaining multiple input signals, measuring a relationship between noise content of the input signals, and combining the input signals in consideration of the measured relationship to produce an output signal having low noise content.

The invention provides improved techniques for measuring T-wave alternans.

The spectral method for T-wave alternans measurement relies on two assumptions. The first assumption is that the physiological T-wave alternans is phase-locked with the T-wave and therefore can be sampled at a constant phase in every beat. This assumption is valid due to the physiological factors associated with the generation of T-wave alternans.

The second assumption is that the noise within the noise reference band is white. To satisfy this condition, colored noise (e.g., motion artifact due to exercise) must be avoided within the noise reference band. During an ergometer exercise test, the pedaling rate can be adjusted to 1/3 or 2/3 of the heart rate using auditory and visual cues to move most of the colored noise away from the noise reference band and the alternans frequency. Nonetheless, colored noise often can fall within the noise reference noise band and at the alternans frequency during ergometer exercise due to the exercise, other motion artifact, respiration, and other sources. Furthermore, during treadmill exercise, it is difficult to control the stepping rate and it is common for the exercise-induced motion artifact to create colored noise that falls within the noise reference band and at the alternans frequency.

In one general aspect, measuring alternans in a physiological signal includes processing the physiological signal to create a processed signal having an asymmetric spectrum (i.e., a spectrum that is asymmetric around DC), and processing the processed signal to measure alternans in the physiologic signal.

Processing the physiological signal to create a processed signal may include creating the processed signal as an analytical signal, or as an approximation of an analytical signal. Creating an analytical signal may include generating a frequency domain representation of the physiological signal, modifying the frequency domain representation to remove components corresponding to negative frequencies, and generating the analytical signal as a time domain representation of the modified frequency domain representation.

The physiological signal may be an electrocardiogram. The electrocardiogram may be recorded from a subject during exercise, such as exercise using an ergometer or a treadmill.

Processing the processed signal may include sampling the processed signal at a frequency less than or equal to twice a frequency corresponding to alternans. For example, the processed signal may be sampled once per beat, where the frequency of alternans is once every other beat. Stated another way, processing involves processing samples of the signal spaced by intervals greater than or equal to half the period of alternans.

In another general aspect, alternans may be measured using a system having an input unit configured to receive the physiological signal, a processor, and an output unit. The processor is connected to the input unit and configured to process the physiological signal to create a processed signal having an asymmetric spectrum, and to process the processed signal to generate an indication of alternans in the physiologic signal. The output unit is connected to the processor and configured to receive and output the indication of alternans.

In another general aspect, a band-limited signal may be analyzed by producing an analytical signal version of the signal, sampling the analytical signal, and processing samples of the signal spaced by intervals greater than or equal to half the period of the highest frequency component of the band-limited signal to produce a sampled analytical signal, and analyzing the sampled analytical signal.

The band-limited signal may be a physiological signal, such as an electrocardiogram, and the analysis may involve measurement of alternans. The band-limited signal also may be an electro-encephalogram signal.

When the band-limited signal is periodic, the analysis may include detecting frequency components which are located at or near submultiples of the reciprocal of the period of the band-limited signal.

Other features and advantages will be apparent from the following description, including the drawings, and from the claims.

Fig. 1 is a graph of an ECG waveform for a single beat.

Figs. 2A and 2B are graphs of, respectively, a band-limited signal and the power spectrum of the signal. Figs. 3A and 3B are graphs of, respectively, the band-limited signal of Fig. 2A sampled at a frequency greater than twice the frequency of the highest frequency component of the band-limited signal, and the corresponding power spectrum for the sampled signal.

Figs. 4A and 4B are graphs of, respectively, the band-limited signal of Fig. 2A sampled at a frequency less than twice the frequency of the highest frequency component of the band-limited signal, and the corresponding power spectrum for the sampled signal.

Fig. 5A is a plot of the heart rate of a patient versus time during a treadmill exercise test; Fig. 5B is plot of the stepping rate of the patient versus time; and Fig. 5C is a plot of the stepping rate divided by the heart rate (solid line) and the first sub-harmonic of the stepping rate divided by the heart rate (dotted line).

Fig. 6 is a flow chart of a procedure for processing ECG signals.

Figs. 7A and 7B are graphs of, respectively, a band-limited signal and the power spectrum of the signal.

Fig. 8 is a graph of the transfer function of a filter used to generate an analytical signal from a band-limited signal.

Fig. 9 is a graph of a power spectrum of the analytical signal.

Figs. 10A and 10B are graphs of, respectively, the analytical signal sampled at a frequency less than twice the frequency of the highest frequency component of the band-limited signal, and the corresponding power spectrum.

Figs. 11A and 11B are graphs of power spectra generated using, respectively, an analytical signal approach and the spectral method.

Techniques are described for processing an ECG signal to reduce or eliminate the effect of colored noise. Detection of altemans in an ECG signal may be improved by processing the ECG signal to reduce or eliminate the effects of noise. However, in processing a signal that includes colored noise, errors may result if one assumes that the noise is white.

Theoretically, to avoid aliasing when sampling a signal at a given rate, $F_s$, the signal must be band limited to half of the sampling frequency, $0.5F_s$, which is referred to as the Nyquist frequency.

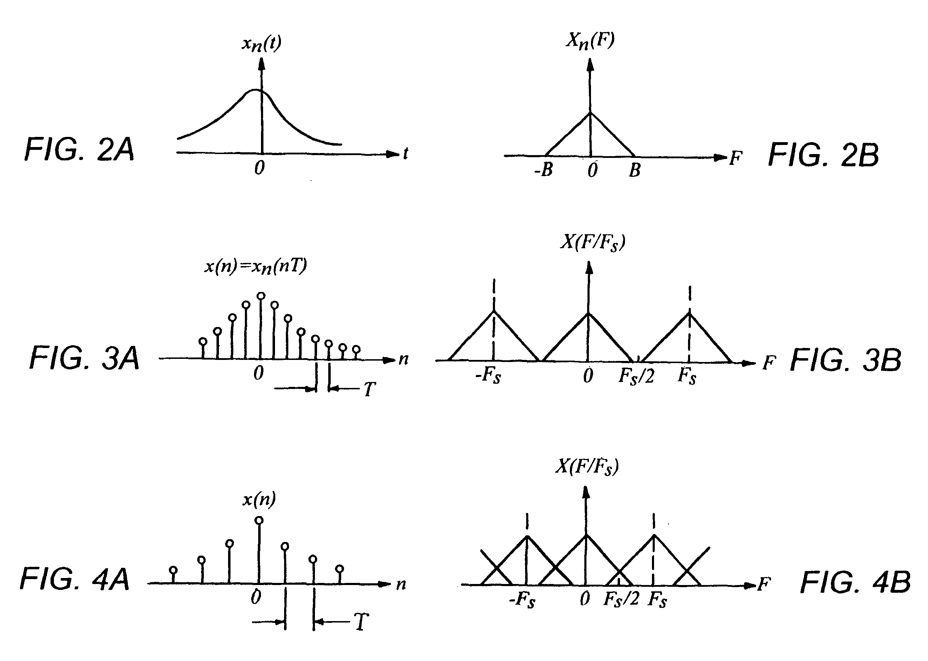

Figs. 2A and 2B show, respectively, a band-limited analog signal $x_a(t)$ and the power spectrum $X_a(f)$ for that signal. Note that the power spectrum is symmetric about zero.

When the analog signal is sampled, the spectrum for the sampled signal is periodic with a period equal to the sampling frequency, $F_s$. Figs. 3A and 3B show a case in which the sampling frequency is greater than twice the signal bandwidth, $2B$. As shown, there is no interference between adjacent spectral periods, and, accordingly, an accurate measurement of signal power at all frequencies of the original analog signal can be made by considering the spectrum for a spectral period.

Figs. 4A and 4B show a case in which the sampling rate is smaller than $2B$. As shown, interference between adjacent spectral periods distorts the spectrum for the frequencies of overlap.

As shown in Figs. 4A and 4B, failure to comply with the Nyquist requirement (i.e., use of a sampling frequency smaller than twice the signal bandwidth) results in underestimation of signal power at all overlapped frequencies including the Nyquist frequency. For alternans detection, the sampling rate is limited to one sample per beat and, since the altemans frequency is at exactly the Nyquist frequency, the signal cannot be band limited to comply with the Nyquist requirement.

The spectral method for T-wave alternans measurement is an accurate method in the case of T-wave alternans measured during exercise tests performed on an ergometer with the pedaling rate well controlled at 1/3 or 2/3 of the heart rate. This is because two conditions tend to reduce or eliminate the effects of failure to comply with the Nyquist requirement.

First, the noise within the noise band can be considered to be white. Since the spectrum for white noise is flat for all frequencies, there is interference from multiple adjacent spectral cycles. This, in turn, means that interference due to noise is statistically equivalent for all frequencies.

Second, as noted above, the alternans is phased-locked (i.e. the ECG signal is sampled at synchronized points). This means that the signals at the Nyquist frequency interfere with consistent phase, which results in a correct estimation of signal power at this frequency.

Colored noise artifacts may occur in T-wave altemans measured during ergometer exercise. For example the pedaling rate may not be well controlled, or the artifact due to respiration may cause colored noise to occur in the noise band and at the alternans frequency.

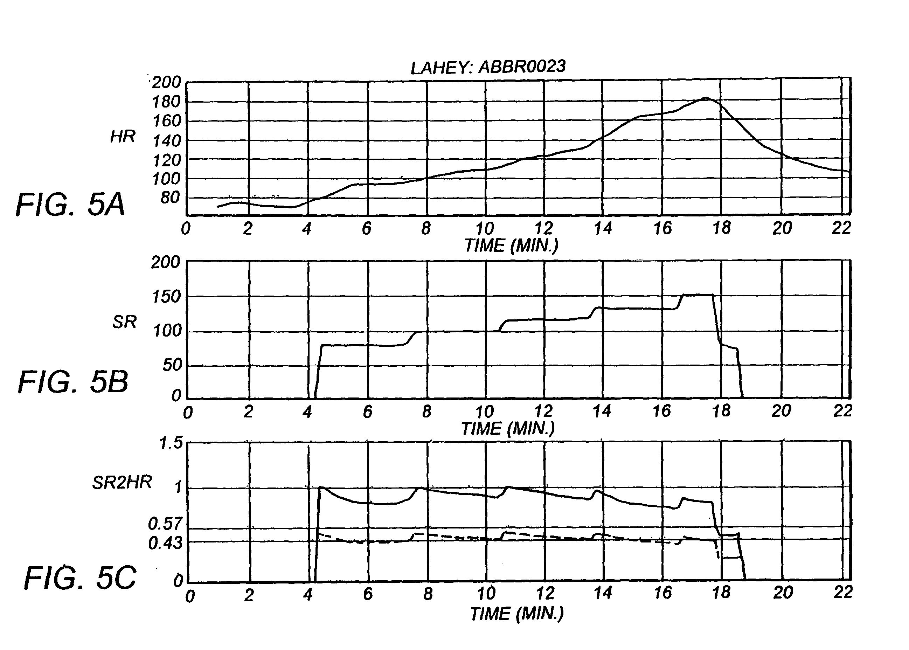

Colored noise may also exist for tests using treadmill exercise. With such exercise, motion artifact due to walking/running may produce unwanted signals of colored nature at or close to the alternans frequency. In treadmill exercise tests, the walking rate is generally close to the heart rate such that sub-harmonics of the heart rate may create significant noise components within the noise band. Figs. 5A-5C show a typical treadmill exercise test case in which the patient's stepping rate is close to the heart rate. Fig. 5A shows the heart rate as a function of time, Fig. 5B shows the stepping rate, and Fig. 5C shows the stepping rate and its sub-harmonic, normalized to the heart rate. In this particular case, the stepping creates artifacts at frequencies close to half of the heart rate.

In a case such as is illustrated in Figs. 5A-5C, since the noise within the noise band is colored, interference between components from adjacent spectra of different phase results in underestimation of noise and therefore overestimation of alternans power, which in turn may produce false positive results for treadmill T-wave alternans tests.

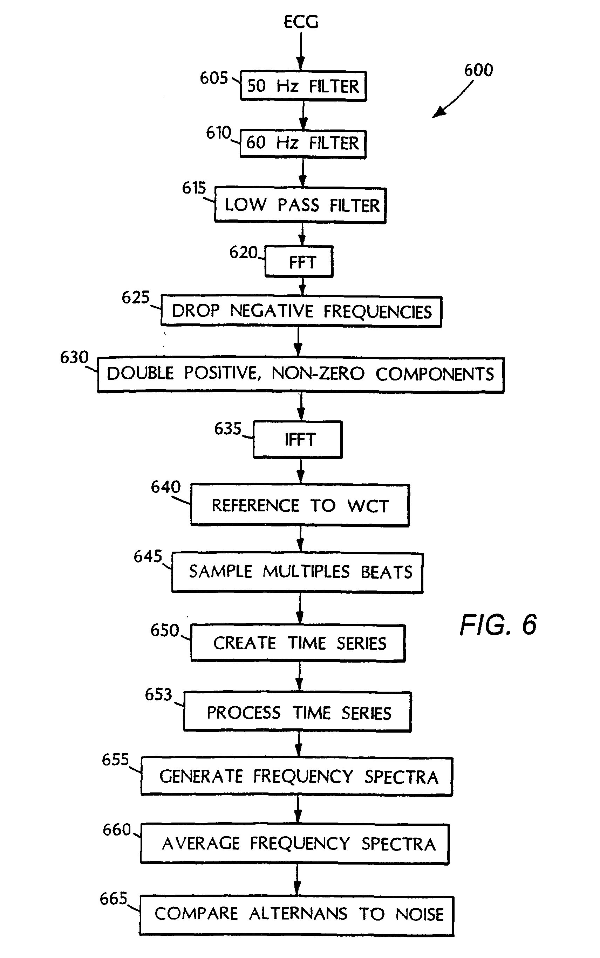

Referring to Fig. 6, problems associated with the presence of colored noise may be avoided through use of an analytical signal technique 600. According to the technique 600, an ECG signal is processed using a 50 Hz filter (step 605) and a 60 Hz filter (step 610). This processing reduces the effects of line voltages used to power the equipment that generates the ECG signal, with 60 Hz being the standard line voltage frequency in the U.S. and 50 Hz being standard in Europe.

Next, an analytical version of the signal is created (steps 615-635). First, the signal is low-pass filtered (step 615). In one implementation, the low pass filter is a 5th order Butterworth filter with a zero phase configuration. The filtered signal is then transferred to the frequency domain using a fast Fourier transform (FFT) (step 620).

In the frequency domain, the portions of the frequency spectrum corresponding to negative frequencies are removed (step 625). The technique then compensates for removal of negative frequencies by doubling all positive, non-zero components of the frequency spectrum (step 630). An inverse fast Fourier transform (IFFT) is then performed on the modified frequency spectrum to produce an analytical signal in the time domain (step 635).

Next, the analytical signal is referenced to an analytical version of Wilson's central terminal (step 640). Wilson's central terminal (WCT) is a well-known ECG reference value. The analytical version of WCT is generated from the standard WCT using the procedure set forth in steps 615-635. The analytical signal is referenced to the analytical version of WCT by determining the difference between the two signals.

The referenced analytical signal then is processed similarly to the spectral method. In particular, the referenced analytical signal is sampled at time synchronized points on the T wave for a collection of 128 beats (step 645), and a time series is created for each point on the collection of T waves (step 650). As in the spectral method, a time series is created by measuring, for each of the 128 beats, the T-wave level at a fixed point relative to the QRS complex. This process is repeated to create a time series for each point in the T wave.

Next, the time series are processed to reduce noise such as that resulting from baseline wander (step 653). In general, this processing uses other signals, including those corresponding to respiration and impedance, to adaptively remove baseline wander. Techniques for processing the time series are described in more detail in U.S. patent 5,704,365, titled "USING RELATED SIGNALS TO REDUCE ECG NOISE,".

A frequency spectrum is then generated for each time series (step 655), and the spectra are averaged to form a composite T-wave alternans spectrum (step 660). Since the T-waves are sampled once per beat for each time series, the spectral value at the Nyquist frequency, i.e. 0.5 cycle per beat, indicates the level of beat-to-beat alternation in the T-wave waveform.

Finally, the alternans power is statistically compared to the noise power to discriminate the beat-to-beat T-wave variation due to abnormal electrical activity of the heart from the random variation due to background noise (step 665). The alternans power is calculated by subtracting the mean power in a reference band used to estimate the background noise level from the power at the Nyquist frequency (0.50 cycle per beat). In one implementation, the reference band includes frequencies from 0.43 to 0.49 and 0.51 to 0.56 cycles per beat. In the same implementation, alternans is considered to be significant if it is at least three times the standard deviation of the noise in the noise reference band.

In general, the technique 600 reduces or eliminates the effects of aliasing. The amount of aliasing depends on the patient's heart rate and reduces as the heart rate increases. For heart rates of primary interest, such as 80 to 120 beats per minute, the sampling frequency is approximately 2 Hz. In the spectral method, this would have meant that any signal component of frequency content over 1 Hz would be a source of aliasing.

Since aliasing is primarily due to the interference between the frequency components at the positive part of the spectrum and those at the negative part of the spectrum from an adjacent period of the spectrum, creation of an analytical signal serves to avoid aliasing. In particular, creation of the analytical signal removes the interfering negative frequency components while scaling the signal to preserve the total signal energy.

An analytical signal is a complex signal. See Proakis JG, Manolakis DG, Digital Signal Processing, Prentice Hall, Upper Saddle River, NJ, 1996, pp. 738-742. The real part of the complex signal, y, is the original signal, x, and the imaginary part is the Hilbert transform, H(x), of the original signal:

$$y = x + jH(x),$$

where $H(x)$ is the Hilbert Transform of $x$ with the following transfer function.

$$H(\omega) = \begin{cases} -j & \text{for} \quad 0 < \omega \leq +n \\ +j & \text{for} \quad -\pi < \omega \leq 0 \end{cases}$$

The Hilbert Transform returns a complex sequence. This sequence is a version of the original real sequence with a 90° phase shift. It has the same amplitude and frequency content as the original real data and includes phase information that depends on the phase of the original data.

The overall transform has the following real transfer function:

$$H_o(\omega) = \begin{cases} 0 & \text{for} \quad -\pi < \omega \leq 0 \\ 1 & \text{for} \quad \omega = 0 \\ 2 & \text{for} \quad 0 < \omega \leq +\pi \end{cases}$$

The analytic signal is characterized as having an asymmetric spectrum with components of negative frequency having been removed. A variety of time domain and frequency domain processing methods and filters that can be used to implement or approximate the analytic signal approach. These methods affect certain frequencies $\omega_n$ of the input signal differently for the positive frequency $+|\omega_n|$ and the corresponding negative frequency $-|\omega_n|$. The result is a signal having an asymmetric spectrum. Examples of suitable processing methods and filters include, but are not limited to, spectral windowing functions and time domain functions which convolve the input signal with a signal whose spectrum is asymmetric. There are a number of techniques that may be used to create suitable functions. These techniques include, but are not limited to, Cbebyshev approximation, FIR or IIR filter design, windowing techniques, recursive design techniques, and inverse Z-transform techniques.

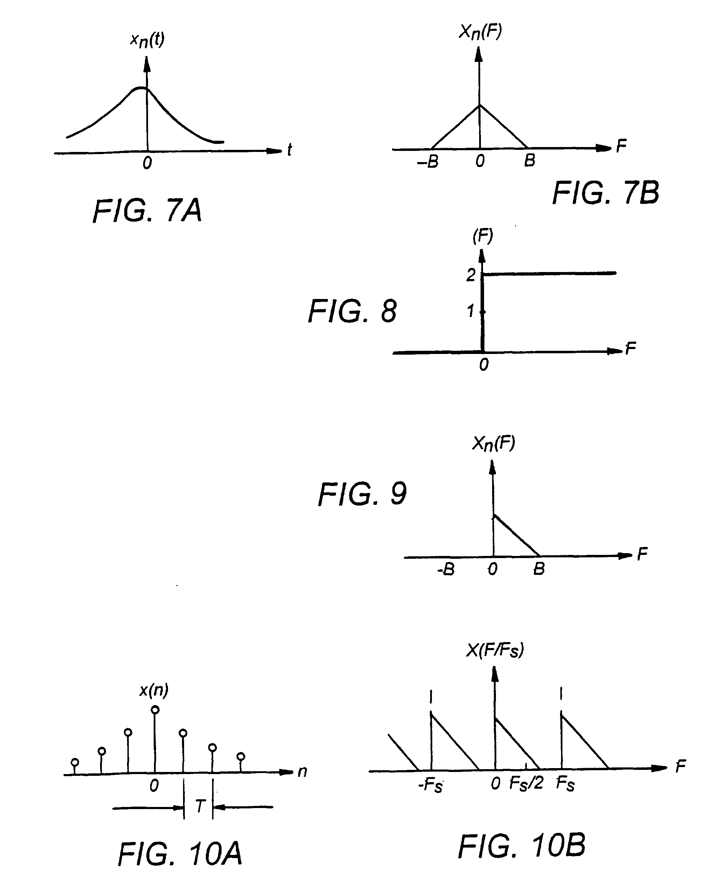

The band-limited signal shown in Fig. 7A has the power spectrum shown in Fig. 7B. When the filter shown in Fig. 8 is applied to the signal of Fig. 7A, an analytical signal having the power spectrum shown in Fig. 9 is created. That signal then may be sampled at a frequency less than twice the bandwidth, as shown in Fig. 10A. For an electrocardiogram signal, by down sampling the signal at T-wave locations, the digital spectrum is still a periodic spectrum with a period of 1/sampling interval, i.e., the heart rate. As shown in Fig. 10B, interference between the positive and negative frequencies is eliminated since the negative part of the spectrum is removed.

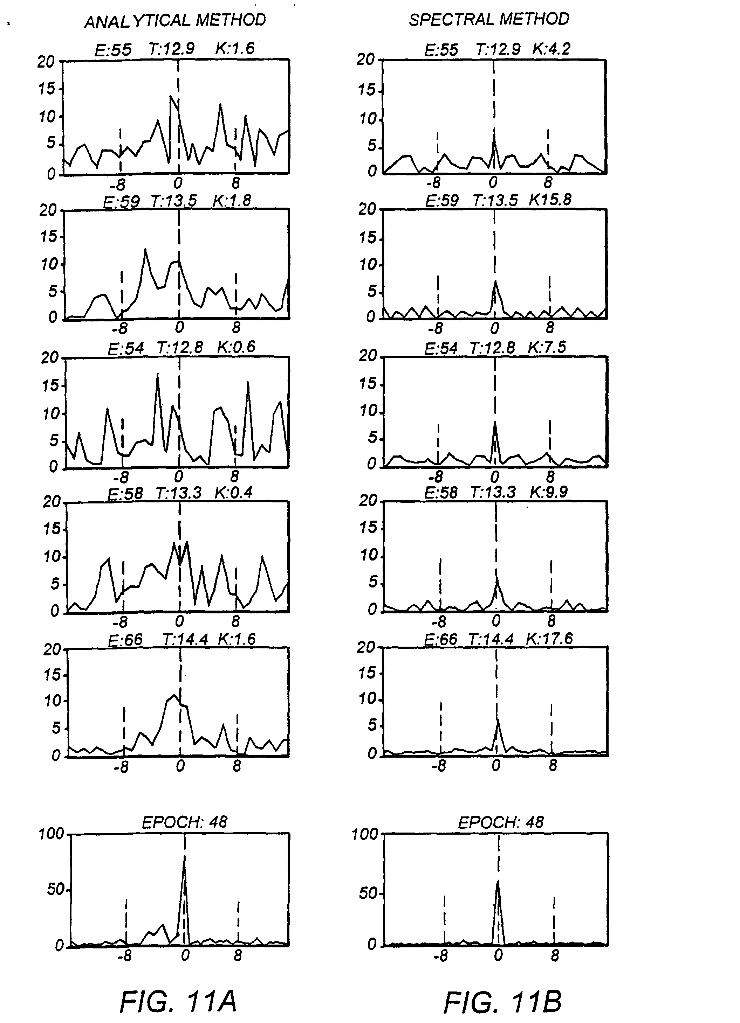

This approach allows an accurate measurement of T-wave alternans even when there is colored noise at or close to alternans frequency, such as may occur in treadmill exercise tests. Figs. 11A and 11B illustrate a comparison between the analytical approach and the existing spectral method. It is evident that the presence of colored noise within the noise band results in an overestimation of alternans power and underestimation of noise power in the spectral method. By contrast, the analytical method provides an accurate estimation of both the altemans and the noise within the noise band.

Other embodiments are within the scope of the following claims.

## Claims

1. A method for measuring alternans in a physiological signal, the method comprising:

   processing the physiological signal to create a processed signal having an asymmetric spectrum; and

   processing the processed signal to measure alternans in the physiologic signal.

2. The method of Claim 1, wherein processing the physiological signal to create a processed signal comprises creating the processed signal as an analytical signal.

3. The method of Claim 2, wherein creating the processed signal as an analytical signal comprises generating a frequency domain representation of the physiological signal, modifying the frequency domain representation to remove components corresponding to negative frequencies, and generating the analytical signal as a time domain representation of the modified frequency domain representation.

4. The method of any one of the preceding claims, wherein the physiological signal comprises an electrocardiogram signal.

5. The method of any one of the preceding claims, wherein the physiological signal comprises an electrocardiogram recorded from a subject during exercise.

6. The method of Claim 5, wherein the exercise comprises using an ergometer or a treadmill.

7. The method of any one of the preceding claims, wherein processing the processed signal includes sampling the processed signal at a frequency less than or equal to twice a frequency corresponding to alternans.

8. The method of any one of Claims 1 to 6, wherein processing the processed signal includes processing samples of the signal spaced by intervals greater than or equal to half the period of alternans.

9. The method of any one of the preceding claims, wherein processing the physiological signal comprises creating an approximation of an analytical signal version of the physiological signal.

10. A system for measuring alternans in a physiological signal, the system comprising:

    an input unit configured to receive the physiological signal;

    a processor connected to the input unit and configured to process the physiological signal to create a processed signal having an asymmetric spectrum, and to process the processed signal to generate an indication of alternans in the physiological signal; and

    an output unit connected to the processor and configured to receive and output the indication of alternans.

11. The system of Claim 10, wherein the processor is configured to create the processed signal as an analytical signal.

12. The system of Claim 11, wherein the processor is configured to create the processed signal as an analytical signal by generating a frequency domain representation of the physiological signal, modifying the frequency domain representation to remove components corresponding to negative frequencies, and generating the analytical signal as a time domain representation of the modified frequency domain representation.

13. The system of Claim 11 or Claim 12, wherein the input unit comprises circuitry configured to receive an electrocardiogram signal, the system further comprising an electrode connected to the input unit and configured to produce an electrocardiogram signal.

14. The system of any one of Claims 10 to 13, wherein the processor is configured to sample the electrocardiogram signal at a frequency of once per beat.

15. The system of any one of Claims 10 to 14, wherein the processor is configured to sample the processed signal at a frequency less than or equal to twice a frequency corresponding to alternans.

**Patentansprüche**

1. Verfahren zur Messung eines Alternans in einem physiologischen Signal, wobei das Verfahren aufweist:

    Verarbeitung des physiologischen Signals, um ein aufbereitetes Signal zu erzeugen, das ein asymmetrisches Spektrum aufweist; und Verarbeitung des aufbereiteten Signals, um den Alternans im physiologischen Signal zu messen.

2. Verfahren nach Anspruch 1, wobei die Verarbeitung des physiologischen Signals, um ein aufbereitetes Signal zu erzeugen, die Erzeugung des aufbereiteten Signals als ein analytisches Signal umfaßt.

3. Verfahren nach Anspruch 2, wobei die Erzeugung des aufbereiteten Signals als ein analytisches Signal die Erzeugung einer Frequenzbereichsdarstellung des physiologischen Signals, das Modifizieren der Frequenzbereichsdarstellung, um Komponenten zu entfernen, die negativen Frequenzen entsprechen, und die Erzeugung des analytischen Signals als eine Zeitbereichsdarstellung der modifizierten Frequenzbereichsdarstellung umfaßt.

4. Verfahren nach einem der vorhergehenden Ansprüche, wobei das physiologische Signal ein Elektrokardiogrammsignat aufweist.

5. Verfahren nach einem der vorhergehenden Ansprüche, wobei das physiologische Signal ein Elektrokardiogramm aufweist, das von einem Patienten während einer Übung aufgezeichnet wird.

6. Verfahren nach Anspruch 5, wobei die Übung die Verwendung eines Ergometers oder eines Laufbands umfaßt.

7. Verfahren nach einem der vorhergehenden Ansprüche, wobei die Verarbeitung des aufbereiteten Signals die Abtastung des aufbereiteten Signals mit einer Frequenz aufweist, die kleiner oder gleich dem Doppelten einer Frequenz ist, die dem Attemans entspricht.

8. Verfahren nach einem der Ansprüche 1 bis 6, wobei die Verarbeitung des aufbereiteten Signals die Verarbeitung von Abtastwerten des Signals aufweist, die in Intervallen beabstandet sind, die größer oder gleich der Hälfte der Periode des Altemans sind.

9. Verfahren nach einem der vorhergehenden Ansprüche, wobei die Verarbeitung des physiologischen Signals die Erzeugung einer Näherung einer analytischen Signalversion des physiologischen Signals aufweist.

10. System zur Messung eines Altemans in einem physiologischen Signal, wobei das System aufweist:

    eine Eingangseinheit, die eingerichtet ist, das physiologische Signal zu empfangen; einen Prozessor, der mit der Eingangseinheit verbunden und eingerichtet ist, das physiologische Signal zu verarbeiten, um ein aufbereitetes Signal zu erzeugen, das ein asymmetrisches Spektrum aufweist, und das aufbereitete Signal zu verarbeiten, um eine Anzeige des Alternans im physiologischen Signal zu erzeugen; und eine Ausgangseinheit, die mit dem Prozessor verbunden ist und eingerichtet ist, die Anzeige des Alternans zu empfangen und auszugeben.

11. System nach Anspruch 10, wobei der Prozessor eingerichtet ist, das aufbereitete Signal als ein analytisches Signal zu erzeugen.

12. System nach Anspruch 11, wobei der Prozessor eingerichtet ist, das aufbereitete Signal als ein analytisches Signal zu erzeugen, durch Erzeugung einer Frequenzbereichsdarstellung des physiologischen Signals, Modifizieren der Frequenzbereichsdarstellung, um Komponenten zu entfemen, die negativen Frequenzen entsprechen, und Erzeugung das analytischen Signals als eine Zeitbereichsdarstellung der modifizierten Frequenzbereichsdarstettung.

13. System nach Anspruch 11 oder 12, wobei die Eingangseinheit einen Schattungskomplex aufweist, der eingerichtet ist, ein Elektrokardiogrammsignal zu empfangen, wobei das System ferner eine Elektrode aufweist, die mit der Eingangseinheit verbunden und eingerichtet ist, ein Elektrokardiogrammsignal zu erzeugen.

**14.** System nach einem der Ansprüche 10 bis 13, wobei der Prozessor eingerichtet ist, das Elektrokardiogrammsignal mit einer Frequenz von einmal pro Schlag abzutasten.

**15.** System nach einem der Ansprüche 10 bis 14, wobei der Prozessor eingerichtet ist, das aufbereitete Signal mit einer Frequenz abzutasten, die kleiner oder gleich dem Doppelten einer Frequenz ist, die dem Alternans entspricht.

**Revendications**

**1.** Procédé de mesure de l'alternans dans un signal physiologique, le procédé comprenant :

le traitement du signal physiologique pour créer un signal traité ayant un spectre asymétrique ; et

le traitement du signal traité pour mesurer l'alternans dans le signal physiologique.

**2.** Le procédé de la revendication 1, dans lequel le traitement du signal physiologique pour créer un signal traité comprend la création du signal traité en tant que signal analytique.

**3.** Le procédé de la revendication 2, dans lequel le traitement du signal traité en tant que signal analytique comprend la génération d'une représentation de domaine de fréquence du signal physiologique, la modification de la représentation de domaine de fréquence pour supprimer les composants correspondant aux fréquences négatives, et la génération du signal analytique en tant que représentation de domaine de temps de la représentation de domaine de fréquence modifiée.

**4.** Le procédé de l'une quelconque des revendications précédentes, dans lequel le signal physiologique comprend un signal d'électrocardiogramme.

**5.** Le procédé de l'une quelconque des revendications précédentes, dans lequel le signal physiologique comprend un électrocardiogramme enregistré sur un sujet pendant un exercice.

**6.** Le procédé de la revendication 5, dans lequel l'exercice comprend l'utilisation d'un ergomètre ou d'un tapis roulant.

**7.** Le procédé selon l'une quelconque des revendications précédentes, dans lequel le traitement du signal traité comprend l'échantillonnage du signal traité à une fréquence inférieure ou égale à deux fois une fréquence correspondant à l'alternans.

**8.** Le procédé selon l'une quelconque des revendications 1 à 6, dans lequel le traitement du signal traité comprend le traitement d'échantillons du signal espacés par des intervalles égaux ou supérieurs à la moitié de la période de l'alternans.

**9.** Le procédé de l'une quelconque des revendications précédentes, dans lequel le traitement du signal physiologique comprend la création d'une approximation d'une version signal analytique du signal physiologique.

**10.** Système de mesure de l'alternans dans un signal physiologique, le système comprenant :

une unité d'entrée configurée pour recevoir le signal physiologique ;

un processeur connecté à l'unité d'entrée et configuré pour traiter le signal physiologique pour créer un signal traité ayant un spectre asymétrique, et pour traiter le signal traité pour générer une indication de l'alternans dans le signal physiologique ; et

une unité de sortie connectée au processeur et configurée pour recevoir et produire l'indication de l'alternans.

**11.** Le système de la revendication 10, dans lequel le processeur est configuré pour créer le signal traité en tant que signal analytique.

**12.** Le système de la revendication 11, dans lequel le processeur est configuré pour créer le signal traité en tant que signal analytique par génération d'une représentation de domaine de fréquence du signal physiologique, modification de la représentation de domaine de fréquence pour supprimer les composants correspondant à des fréquences négatives, et génération du signal analytique en tant que représentation de domaine de temps de la représentation de domaine de fréquence modifiée.

**13.** Le système de la revendication 11 ou de la revendication 12, dans lequel l'unité d'entrée comprend des circuits configurés pour recevoir un signal d'électrocardiogramme, le système comprenant en outre une électrode connectée à l'unité d'entrée et configurée pour produire un signal d'électrocardiogramme.

**14.** Le système de l'une quelconque des revendications 10 à 13, dans lequel le processeur est configuré pour échantillonner le signal d'électrocardiogramme à une fréquence d'une fois par battement.

**15.** Le système de l'une quelconque des revendications 10 à 14, dans lequel le processeur est configuré pour échantillonner le signal traité à une fréquence égale ou inférieure à deux fois une fréquence correspondant à l'alternans.

FIG. 1

FIG. 2A
FIG. 2B

$x_n(t)$
$X_n(F)$

FIG. 3A
FIG. 3B

$x(n) = x_n(nT)$
$X(F/F_s)$

FIG. 4A
FIG. 4B

$x(n)$
$X(F/F_s)$

FIG. 5A

LAHEY: ABBR0023

FIG. 5B

FIG. 5C

EP 1 215 996 B1

ECG

605 — 50 Hz FILTER

600

610 — 60 Hz FILTER

615 — LOW PASS FILTER

620 — FFT

625 — DROP NEGATIVE FREQUENCIES

630 — DOUBLE POSITIVE, NON-ZERO COMPONENTS

635 — IFFT

640 — REFERENCE TO WCT

645 — SAMPLE MULTIPLES BEATS

650 — CREATE TIME SERIES

*FIG. 6*

653 — PROCESS TIME SERIES

655 — GENERATE FREQUENCY SPECTRA

660 — AVERAGE FREQUENCY SPECTRA

665 — COMPARE ALTERNANS TO NOISE

$x_n(t)$

FIG. 7A

$X_n(F)$

$-B$  0  $B$  $F$

FIG. 7B

$(F)$

2

1

0  $F$

FIG. 8

$X_n(F)$

FIG. 9

$-B$  0  $B$  $F$

$x(n)$

0

$T$

$n$

FIG. 10A

$X(F/F_s)$

$-F_s$  0  $F_s/2$  $F_s$  $F$

FIG. 10B

ANALYTICAL METHOD

SPECTRAL METHOD

FIG. 11A

FIG. 11B